Europäisches Patentamt

European Patent Office

Office européen des brevets

(19)

(11) Veröffentlichungsnummer: **0 185 249**
**B1**

(12) **EUROPÄISCHE PATENTSCHRIFT**

(45) Veröffentlichungstag der Patentschrift: **03.10.90**

(21) Anmeldenummer: **85115381.7**

(22) Anmeldetag: **04.12.85**

(51) Int. Cl.⁵: **C 07 C 33/22, C 07 C 29/82**

(54) **Verfahren zur Reinigung von rohem beta-Phenylethylalkohol.**

(30) Priorität: **19.12.84 DE 3446265**

(43) Veröffentlichungstag der Anmeldung:
**25.06.86 Patentblatt 86/26**

(45) Bekanntmachung des Hinweises auf die
Patenterteilung:
**03.10.90 Patentblatt 90/40**

(84) Benannte Vertragsstaaten:
**CH DE FR GB LI NL**

(56) Entgegenhaltungen:
**BE-A- 676 588**
**FR-A-1 301 176**
**FR-A-2 467 836**
**GB-A- 923 464**

(73) Patentinhaber: **Haarmann & Reimer GmbH**
**Postfach 1253**
**D-3450 Holzminden (DE)**

(72) Erfinder: **Nienhaus, Jürgen, Dr.**
**Hoher Weg 7c**
**D-3450 Holzminden (DE)**
Erfinder: **Hopp, Rudolf, Dr.**
**Auf dem Gehrenkamp 28**
**D-3450 Holzminden (DE)**

(74) Vertreter: **Schumacher, Günter, Dr. et al**
**c/o Bayer AG Konzernverwaltung RP**
**Patentabteilung**
**D-5090 Leverkusen 1 Bayerwerk (DE)**

**Beschreibung**

Die Erfindung betrifft ein neues Verfahren zur Reinigung von rohem ß-Phenylethylalkohol durch Azeotrop-Destillation.

Phenylethylalkohol ist ein preisgünstiger Riechstoff mit mildem Rosengeruch. Sein Anteil in Riechstoffkompositionen kann 5 bis 20 Gew.-% und manchmal auch noch mehr als 20 Gew.-% betragen. Wegen seines milden Geruches und der relativ hohen Einsatzmengen werden hohe Anforderungen an die Reinheit dieses Riechstoffes gestellt.

Roher ß-Phenylethylalkohol wird industriell in größerem Maßstab als Nebenprodukt bei der nicht-katalytischen Luftoxidation von Ethylbenzol und bei der Epoxydation olefinisch ungesättigter Verbindungen z.B. Propylen, mittels Ethylbenzolhydroperoxid gewonnen. Der auf diese Weise erhaltene rohe ß-Phenylethylalkohol läßt sich durch Destillation nicht soweit reinigen, daß er als Riechstoff verwendbar ist. Um zu einem geruchlich zufriedenstellenden Produkt zu gelangen, muß der Roh-ß-Phenylethylalkohol besonderen Reinigungsverfahren unterworfen werden. Gemäß US-Patentschrift 4 400 558 wird vorgeschlagen, ein unlösliches Metallhalogenid-ß-Phenylethylalkohol-Addukt, vorzugsweise ein Calciumchlorid-Phenylethylalkohol-Addukt herzustellen, dieses abzutrennen und daraus den Alkohol freizusetzen. Die Reinheit des auf diese Weise erhaltenen ß-Phenylethylalkohols ist zwar durchaus befriedigend, das Verfahren aber sehr aufwendig.

In der US-PS 4,359,365 wird die Reinigung durch Destillation des rohen ß-Phenylethylalkohols in Gegenwart von Wasser, Glycerin, einem Alkylenglykol, einen Polyalkylenglykol, einem Alkylenglykolmonomethylmonoalkylether, einem Polyalkylenglykolmonoalkylether oder einem Gemisch aus mindestens zwei dieser Verbindungen beschrieben. Wird die Destillation in Gegenwart eines Lösungsmittels mit einem höheren Siedepunkt als dem des ß-Phenylethylalkohols durchgeführt, handelt es sich um eine extraktive Destillation, liegt der Siedepunkt des Lösungsmittels tiefer als der des ß-Phenylethylalkohols, handelt es sich um eine azeotrope Destillation. Das Verfahren liefert zwar ß-Phenylethylalkohol in der gewünschten Reinheit, aber da die Selektivität der als azeotropes bzw. extraktives Lösungsmittel zu verwendenden Verbindungen unzureichend ist, ist die Destillation mit erheblichen Verlusten an ß-Phenylethylalkohol verbunden (es gehen etwa 15 bis 20 Gew.-% des im Rohprodukt enthaltenen ß-Phenylethylalkohols verloren).

Überraschenderweise wurde jetzt gefunden, daß bei der Verwendung bestimmter Alkanolamine als a zeotrope Lösungsmittel für die Destillation von Roh-ß-Phenylethylalkohol nicht nur ein ß-Phenylethylalkohol der gewünschten Reinheit erhalten wird, sondern daß diese Alkanolamine zugleich eine wesentlich höhere Selektivität aufweisen als die in der US-PS 4,359,365 als Hilfslösungsmittel für die Destillation empfohlenen Verbindungen und daß deshalb die Destillationsverluste an ß-Phenylethylalkohol auf unter 5 Gew.-% gesenkt werden können.

Die Erfindung betrifft daher ein Verfahren zur Reinigung von rohem ß-Phenylethylalkohol durch Azeotrop-Destillation, das dadurch gekennzeichnet ist, daß der rohe ß-Phenylethylalkohol in Gegenwart von Alkanolaminen destilliert wird, deren Alkylenkette 2 bis 4 Kohlenstoffatome aufweist und gegebenenfalls durch 1 bis 4 $C_1$—$C_3$-Alkylgruppen substituiert ist.

Ethanolamin wurde bereits als Hilfsmittel für die Azeotrop-Destillation von Kohlenwasserstoffen (siehe Zh. Prikl. Khim. (Leningrad) *1980*, 53 (10), 2388; CA *93*, (1980) 246446 k; und Japan. Kokai 72/16246; CA *77*, (1972) 139586 d) und für die Reinigung von B-brazan (Benzo-[b]-naphtho-[2,3-d]-furan) (siehe Koks. Khim. 1968 (1) 41—43; CA *69*, (1968) 70459 e) vorgeschlagen. Da aber in diesen Veröffentlichungen Ethanolamin gleichwertig neben Ethylenglykolen, Polyethylenglykolen, Alkylaminen und Alkylendiaminen genannt wird, und diese Hilfslösungsmittel wegen ihrer unzureichenden Selektivität für die Azeotrop-Destillation von ß-Phenylethylalkohol ungeeignet sind, war aus diesen Veröffentlichungen in keiner Weise die besondere Eignung der erfindungsgemäß zu verwendenden Aminoalkohole, insbesondere des 2-Amino-ethanols, für die Azeotrop-Destillation von ß-Phenylethylalkohol vorauszusehen.

Die erfindungsgemäß als Hilfslösungsmittel in der Azeotrop-Destillation des ß-Phenylethylalkohols einzusetzenden Alkanolamine haben einen Siedepunkt, der unter dem des ß-Phenylethylalkohols (219,8°C) liegt. Solche Alkanolamine sind z.B. 2-Aminoethanol, 1-Aminopropanol-(2), 2-Aminopropanol-(1), 2-Amino-butanol-(1), 1-Amino-butanol-(2), 2-Amino-pentanol-(1), 3-Amino-pentanol-(2), 1-Amino-3-methyl-butanol-(2), 2-Amino-2-methyl-propanol-(1), 3-Amino-propanol-(1), 2-Amino-butanol-(3), 3-Amino-butanol-(1), 1-Amino-3-methyl-butanol-(3), 1-Amino-2,2-dimethyl-propanol-(3), 2-Amino-3-methyl-hexanol-(4), 2-Amino-2,4-dimethyl-pentanol-(4) und 4-Amino-butanol-(1). Bevorzugt verwendete Alkanolamine sind 2-Aminoethanol, 1-Amino-propanol-(2) und 3-Amino-propanol-(1).

Die Menge an Alkanolamin ist in einem weiten Bereich variierbar; bewährt hat sich, die Alkanolamine in einer Menge von 0,1 bis 10 Gew.-Teilen, vorzugsweise 0,1 bis 1 Gew.-Teilen je Gew.-Teil rohen ß-Phenylethylalkohols einzusetzen.

Das erfindungsgemäße Verfahren kann kontinuierlich oder diskontinuierlich durchgeführt werden. Im letzteren Fall werden roher ß-Phenylethylalkohol und das Alkanolamin vorgelegt und in einer vielbödigen Kolonne einer fraktionierten Destillation unterworfen. Am Kolonnenkopf werden als erste Fraktion die Verunreinigungen zusammen mit dem Alkanolamin abgenommen. Dann folgt eine kleine Zwischenfraktion, die neben dem Alkanolamin bereits etwas ß-Phenylethylalkohol enthält und bei der nächsten Destillation wieder eingesetzt werden kann; abschließend wird parfümistisch befriedigender ß-Phenylethylalkohol abgenommen. Die Destillation wird unter für die Destillation von ß-Phenylethylalkohol üblichen Bedingungen, d.h. entweder unter Normaldruck oder vermindertem Druck von 1 bis 1000 mbar und bei einer Temperatur von 50 bis 220°C, vorgenommen. Vorzugsweise wird ein Rückflußverhältnis von 0,1:1 bis 50:1, vorzugsweise 1:1 bis 10:1 eingehalten.

Bei der kontinuierlichen Destillation werden der rohe ß-Phenylethylalkohol und das Alkanolamin in die Mitte einer Azeotrop-Destillationskolonne eingespeist. Das Azeotrop aus Alkanolamin und Verunreinigungen wird am Kopf der Kolonne, der gereinigte ß-Phenylethylalkohol am Sumpf der Kolonne abgenommen. Der so gereinigte ß-Phenylethylalkohol wird anschließend noch einer zweiten fraktionierten Destillation unterworfen. Die Destillationsbedingungen (Druck, Temperatur, Rücklaufverhältnis) entsprechen den vorstehend für die diskontinuierliche Destillation angegebenen Bedingungen.

Beispiel 1

600 g roher ß-Phenylethylalkohol, der ewta 8 bis 10 Gew.-% Verunreinigungen enthält, und 200 g 2-Aminoethanol werden gemischt und über eine 1 m-Füllkörperkolonne unter Einhaltung eines Rücklaufverhältnisses von 10:1 bei einem Druck von 100 mbar destilliert.

Der Vorlauf beträgt 194 g; er geht bei einer Sumpftemperatur von 129 bis 150°C und einer Kopftemperatur von 110°C über. Gemäß gaschromatographischer Analyse besteht er aus 89 Gew.-% 2-Aminoethanol und 11 Gew.-% Verunreinigungen. Er enthält keinen ß-Phenylethylalkohol.

Anschließend gehen bei einer Sumpftemperatur von 151°C und einer Kopftemperatur von 110 bis 145°C 80 g einer Zwischenfraktion über. Gemäß gaschromatographischer Analyse besteht diese Zwischenfraktion aus 33 Gew.-% 2-Aminoethanol, 62 Gew.-% ß-Phenylethylalkohol und 5 Gew.-% Verunreinigungen.

Anschließend an diese Zwischenfraktion geht der Hauptlauf (482 g) über. Die Sumpftemperatur steigt dabei bis auf 180°C; die Kopftemperatur beträgt 145°C. Gemäß gaschromatographischer Analyse besteht der Hauptlauf zu 99,9 Gew.-% aus ß-Phenylethylalkohol. Dieser ß-Phenylethylalkohol ist geruchlich einwandfrei und als Riechstoff verwendbar.

Im Sumpf verbleiben 41 g Destillationsrückstand.

Beispiel 2

Es wird wie in Beispiel 1 beschrieben destilliert nur wird anstelle der 200 g 2-Aminoethanol die gleiche Menge an 3-Amino-propanol-(1) verwendet. Es werden 185 g Vorlauf erhalten; er geht bei einer Sumpftemperatur von 125 bis 150°C und einer Kopftemperatur von 120°C über. Gemäß gaschromatographischer Analyse besteht er aus 89 Gew.-% 3-Aminopropanol und 11 Gew.-% Verunreinigungen. Die Menge der Zwischenfraktion beträgt 85 g; sie geht bei einer Sumpftemperatur von 150°C und einer Kopftemperatur von 120 bis 145° über. Sie besteht gemäß gaschromatographischer Analyse aus 36 Gew.-% 3-Aminopropanol, 60 Gew.-% ß-Phenylethylalkohol und 4 Gew.-% Verunreinigungen. Die Hauptfraktion beträgt 475 g; sie geht bei einer Sumpftemperatur von 145 bis 180°C und einer Kopftemperatur von 150°C über. Gemäß gaschromatographischer Analyse besteht die Hauptfraktion zu 99,9 Gew.-% aus ß-Phenylethylalkohol; der ß-Phenylethylalkohol ist geruchlich einwandfrei und als Riechstoff verwendbar. Der Destillationsrückstand beträgt 49 g.

Beispiel 3

Es wird wie in Beispiel 1 beschrieben destilliert nur wird anstatt der 200 g 2-Aminoethanol die gleiche Menge an 1-Amino-propanol-(2) verwendet.

Es werden 190 g Vorlauf erhalten; er geht bei einer Sumpftemperatur von 130 bis 150°C und einer Kopftemperatur von 96 bis 100°C über. Gemäß gaschromatographischer Analyse besteht er aus 90 Gew.-% 1-Amino-propanol-(2) und 10 Gew.-% Verunreinigungen.

Die Menge der Zwischenfraktion beträgt 90 g; sie geht bei einer Sumpftemperatur von 150°C und einer Kopftemperatur von 100 bis 145°C über. Gemäß gaschromatographischer Analyse besteht sie aus 32 Gew.-% 1-Amino-propanol-(2), 59 Gew.-% ß-Phenylethylalkohol und 9 Gew.-% Verunreinigungen. Die Hauptfraktion beträgt 470 g; sie geht bei einer Sumpftemperatur von 150 bis 180°C und einer Kopftemperatur von 145°C über. Gemäß gaschromatographischer Analyse besteht sie zu 99,9 Gew.-% aus ß-Phenylethylalkohol. Der ß-Phenylethylalkohol ist geruchlich einwandfrei und ohne weitere Reinigung als Riechstoff verwendbar.

Der Destillationsrückstand beträgt 43 g.

**Patentansprüche**

1. Verfahren zur Reinigung von rohem ß-Phenylethylalkohol durch Azeotrop-Destillation, dadurch gekennzeichnet, daß der rohe ß-Phenylethylalkohol in Gegenwart von Alkanolaminen destilliert wird, deren Alkylenkette 2 bis 4 Kohlenstoffatome aufweist und gegebenenfalls durch 1 bis 4 $C_1$—$C_3$-Alkylgruppen substituiert ist.

2. Verfahren nach Anspruch 1, dadurch gekennzeichnet, daß die Alkanolamine in einer Menge von 0,1 bis 10 Gew.-Teilen je Gew.-Teile rohen ß-Phenylethylalkohols eingesetzt werden.

3. Verfahren nach Anspruch 1 oder 2, dadurch gekennzeichnet, daß als Alkanolamin 2-Aminoethanol verwendet wird.

**Revendications**

1. Procédé de purification d'alcool ß-phényléthylique brut par distillation azéotropique, caractérisé en ce qu'on distille l'alcool β-phénylé-thylique brut en présence d'alcanolamines dont la chaîne alkylénique présente 2 à 4 atomes de carbone et est substituée le cas échéant par 1 à 4 groupes alkyle en $C_1$ à $C_3$.

2. Procédé suivant la revendication 1, caractérisé en ce que les alcanolamines sont utilisées en une quantité de 0,1 à 10 parties en poids par partie en poids d'alcool β-phényléthylique brut.

3. Procédé suivant la revendication 1 ou 2, caractérisé en ce qu'on utilise comme alcanolamine le 2-aminoéthanol.

**Claims**

1. Process for the purification of crude β-phenylethyl alcohol by azeotropic distillation, characterized in that the crude β-phenylethyl alcohol is distilled in the presence of alkanolamines in which the alkylene chain has 2 to 4 carbon atoms and is optionally substituted by 1 to 4 $C_1$—$C_3$-alkyl groups.

2. Process according to Claim 1, characterized in that the alkanolamines are employed in an amount of 0.1 to 10 parts by weight per part by weight of crude β-phenylethyl alcohol.

3. Process according to Claim 1 or 2, characterized in that 2-aminoethanol is used as the alkanolamine.